# EUROPÄISCHE PATENTSCHRIFT

(11) **EP 1 899 270 B1**
(45) Veröffentlichungstag und Bekanntmachung des Hinweises auf die Patenterteilung: **27.02.2019**
(21) Anmeldenummer: 05753905.8
(22) Anmeldetag: 21.06.2005
(51) Int. Cl.: C01G 49/06, A61K 33/26, B01J 20/06, C02F 1/28, B01J 20/02, A23L 33/16, A23K 20/20

(54) **VERFAHREN ZUR HERSTELLUNG EINES PHOSPHATADSORBENS AUF EISENSULFAT-BASIS**
METHOD FOR PRODUCING AN IRON SULFATE-BASED PHOSPHATE ADSORBENT
ADSORBANT DE PHOSPHATE A BASE DE SULFATE DE FER

(30) Priorität: 28.06.2004 DE 102004031181
(43) Veröffentlichungstag der Anmeldung: 19.03.2008
(73) Patentinhaber: VIFOR (INTERNATIONAL) AG, 9001 St. Gallen (CH)
(72) Erfinder: MÜLLER, Hans-Martin, CH-9032 Engelburg (CH); PHILIPP, Erik, CH-9303 Wittenbach (CH); GEISSER, Peter, CH-9014 St. Gallen (CH)
(74) Vertreter: Gille Hrabal
(86) Internationale Anmeldenummer: PCT/EP2005/052861
(87) Internationale Veröffentlichungsnummer: WO 2006/000547

(56) Entgegenhaltungen:
- WO-A-97/22266
- WO-A-99/15189
- DE-A1- 10 115 415
- DE-A1- 10 232 069
- DE-B- 1 206 874
- US-A- 4 970 079
- US-A- 5 411 569
- R.M. Cornell, U. Schwertmann: "The Iron Oxides: Structure, Properties, Reactions, Occurrence and Uses", 1996, VCH, Weinheim, New York, Basel, Cambridge, Tokyo ISBN: 3-527-28576-8 pages 18-20,

## Beschreibung

Die vorliegende Erfindung betrifft ein Verfahren zur Herstellung einer neuen Zusammensetzung, sowie die Verwendung der Zusammensetzung als Phosphatadsorbens, insbesondere zur Verabreichung bei Menschen oder Tieren.

Aus der EP 0 868 125 ist ein Adsorbens für Phosphat aus wässrigen Medien, das durch Kohlehydrate und/oder Humussäure stabilisiertes polynukleares β-Eisenhydroxid enthält, bekannt. Das Produkt wird durch Umsetzung einer Eisen(III)-chloridlösung mit einer Base (insbesondere Sodalösung) und Zugabe des Kohlehydrates bzw. der Humussäure vor der Alterung des gebildeten Eisenhydroxids hergestellt. Die Verwendung der Eisen(III)-chloridlösung bei der Fällung ist erforderlich, da die Anwesenheit von Chloridionen für die Bildung des β-Eisenhydroxids (Akaganeit) wesentlich ist. Es wird angenommen, dass durch die Zugabe des Kohlehydrates bzw. der Humussäure eine Stabilisierung des frisch hergestellten β-Eisenhydroxids erfolgt, wodurch das erhaltene Material gegenüber einer Mischung von gealtertem β-Eisenhydroxids mit Kohlehydraten bzw. Humussäure ein überlegenes Phosphatabsorptionsvermögen aufweist.

Die Verwendung von Eisen(III)-chlorid als Ausgangsmaterial bei der Herstellung des Phosphatadsorbens gemäß EP 0 868 125 wirft jedoch Probleme auf. So führt die Verwendung des Eisen(III)-chlorids zu Problemen der Korrosion der Anlagen durch die Anwesenheit von Chloridionen. Außerdem ist der Preis für Eisen(III)-chlorid relativ hoch.

WO 97/22266 offenbart ein Adsorbens für Phosphat aus wässerigen Medien, das Kohlenhydrate (Dextrin, Saccharose oder eine Mischung davon) und/oder durch Humussäure stabilisiertes polynukleares Eisenhydroxid enthält, welches durch Umsetzung einer Eisen(III)chlorid-Lösung mit einer Base und Zugabe des Kohlenhydrats und der Humussäure erhalten wird. Die Reaktion erfolgt bei einem pH-Wert von 3 bis 10 und di erhaltene Zusammensetzung enthält bis zu 40 Gew.-% Eisen.

DE 12 06 874 A offenbart ein Verfahren zur Herstellung von weitgehend sulfatfreiem Eisenoxydhydrat, das als Ausgangsmaterial für die Ferritherstellung dient, das aus einer Eisen(II)sulfat, Eisen(II)nitrat oder Eisenchlorid enthaltenden wässerigen Lösung unter Luftzufuhr mittels einer alkalischen Lösung ausgefällt wird.

DE 102 32 069 A1 offenbart ein Verfahren zur Herstellung von Eisenhydroxid, Eisenoxidhydrat oder Eisenoxid aus Filtersalzen der Dünnsäure-Rückgewinnung, bei dem die Filtersalze in Wasser gelöst werden, die Lösung auf einen pH von eingestellt wird, anschließend durch Zugabe einer starken Base auf einen pH von 2 bis 4 eingestellt wird, die dabei ausfallenden Stoffe abgetrennt werden die verbleibende Lösung unter Zugabe einer starken Base und eines Oxidationsmittels auf pH 6 bis 8 eingestellt wird, das ausfallende Eisenhydroxid aus der Lösung abgetrennt, gewaschen, getrocknet und gegebenenfalls zu Eisenoxid geglüht wird.

US 4,970,079 offenbart die Verwendung von Goethit und Ferrihydrit als Phosphatadsorber zur Kontrolle der Serumphosphatspiegel. Beide Modifikationen werden aus Eisen(III)nitrat hergestellt. Die Herstellung erfolgt aus Eisen(III)nitratlösungen durch Zusatz von KOH. Die erhaltene Lösung wird 4 Tage gegen entmineralisiertes Wasser dialysiert. Die Oberfläche der Oxy-Eisenverbindung beträgt bevorzugt mindestens 50 m²/g. Die offenbarte Phosphatadsorptionskapazität des Eisenoxyhydroxids beträgt lediglich 0,05 mg P/mg Fe.

R.M. Cornell, U. Schwertmann "The Iron Oxides: Structure, Properties, Reactions, Occurrence and Uses", 1996, VCH, Weinheim, New York, Basel, Cambridge, Tokyo, ISBN 3-527-28576-8, S. 18-20 ist ein Standardlehrbuch der Eisenoxide und erläutert die verschiedenen Modifikationen von Eisenoxyhydroxiden. Es gibt an, die Akaganeit-Striktur werde durch Chloridionen stabilisiert, ein Teil der Chloridionen könne zwar ausgewaschen werden, die vollständige Entfernung der Chloridionen führe jedoch zur Umwandlung in Goethit oder Hämatit in Abhängigkeit vom pH.
Es bestand daher der Wunsch, ein Phosphatadsorbens bereitzustellen, das die oben beschriebenen Nachteile aufgrund der Verwendung des Eisen(III)-chlorids als Ausgangsmaterials nicht aufweist. Gleichzeitig sollte das Phosphatadsorbens im Wesentlichen das Phosphatadsorptionsvermögen des Materials gemäß EP 0 868 125 aufweisen.
Die vorliegenden Erfinder fanden nun überraschend, dass unter Verwendung von Eisensulfat- und/oder Eisennitrat-Verbindungen als Ausgangsmaterial ein offenbar ebenfalls beispielsweise durch Kohlenhydrate bzw. Humussäuren stabilisiertes Eisenhydroxid ohne die Verwendung von Eisen(III)-Chlorid erhalten werden kann, das in seinem Phosphatadsorptionsvermögen im Wesentlichen dem des Materials der EP 0 868 125 entspricht. Basierend auf diesem Befund vervollständigten die Erfinder die vorliegende Patentanmeldung.
Gegenstand der Erfindung ist somit ein Verfahren zur Herstellung einer Zusammensetzung, die weniger als 0,05 Gew.-% Chlorid und mindestens 20 Gew.-% aber nicht mehr als 50 Gew.-% Eisen enthält, das die folgenden Schritte umfasst:
a) Versetzen einer wässrigen, sulfat- und/oder nitrathaltigen Eisen(III)-salz-Lösung mit mindestens einer Base unter Bildung eines Niederschlags von Eisenhydroxid, wobei ein pH-Wert von mindestens 3 eingestellt wird,
b) Gegebenenfalls ein- oder mehrmaliges Waschen des erhaltenen Niederschlags mit Wasser, wobei eine wässrige Suspension des Eisenhydroxids erhalten wird,
c) Versetzen der erhaltenen wässrigen Suspension mit mindestens einem weiteren Bestandteil, ausgewählt aus Kohenhydraten und Humussäure, der die Alterung des in Schritt b) erhaltenen Niederschlags von Eisenhydroxid inhibiert,
d) Trocknen der im Schritt c) erhaltenen Zusammensetzung.

Im Schritt a) wird eine wässrige, sulfat- und/oder nitrathaltige Eisen(III)-salz-Lösung mit mindestens einer Base unter Bildung eines Niederschlags von Eisenhydroxid umgesetzt, wobei ein pH-Wert von mindestens 3 eingestellt wird.

Bei der wässrigen, sulfathaltigen Eisen(III)-salz-Lösung kann es sich insbesondere um Lösungen von Eisen(III)-sulfat (Fe₂(SO₄)₃) (einschließlich seiner Hydrate) in Wasser handeln. Es können jedoch auch andere wässrige, sulfathaltige Eisen(III)-salz-Lösungen verwendet werden, wie Lösungen von Eisenalaunen, wie KFe(SO₄)₂ oder NH₄Fe(SO₄)₂. Weiterhin ist erfindungsgemäß auch möglich, schwefelsäure-haltige Lösungen von Eisen(II)-sulfat, die der Oxidation beispielsweise mit Salpetersäure unterworfen werden, zu verwenden.

Die verwendete wässrige, sulfathaltige Eisen(III)-salz-Lösung besitzt bevorzugt eine Konzentration von etwa 3 bis 16 Gew.-%, bezogen auf die Menge des Eisens.

Bei der wässrigen, nitrathaltigen Eisen(III)-salz-Lösung kann es sich insbesondere um Lösungen von Eisen(III)-nitrat (Fe(NO₃)₃) (einschließlich seiner Hydrate) in Wasser handeln.

Die verwendete wässrige, nitrathaltige Eisen(III)-salz-Lösung besitzt bevorzugt eine Konzentration von etwa 3 bis 16 Gew.-%, bezogen auf die Menge des Eisens.

Die Menge der zugesetzten Base in Schritt a) wird so gewählt, dass sich ein pH-Wert von mindestens 3 bevorzugt von mindestens etwa 6 eingestellt. Zweckmäßig setzt man so viel Base ein, um eine unter wirtschaftlichen Gesichtspunkten möglichst vollständige Fällung des Eisens aus der Lösung zu erzielen. Im Allgemeinen arbeitet man daher bei pH-Werten von maximal etwa 10. Höhere pH-Werte sind unter wirtschaftlichen Gesichtspunkten nicht mehr sinnvoll. Bevorzugt liegt der pH-Wert, der sich in Schritt a) einstellt, daher bei 3 bis 10, bevorzugter bei etwa 5 bis 8.

Bevorzugt wird als Base in Schritt a) eine Alkalimetall- und/oder Erdalkalimetall-Verbindung verwendet. Besonders bevorzugt handelt es sich um Hydroxide oder Carbonate von Alkali- oder Erdalkalimetallen. Noch bevorzugter sind Alkalicarbonate, Alkalibicarbonate und Alkalimetallhydroxide, insbesondere von Natrium. Die Basen werden zweckmäßig und bevorzugt in Form einer wässrigen Lösung, bevorzugt mit einer Molarität von etwa 0,01 bis 2 mol/L verwendet. Es ist jedoch auch möglich, die Basen in fester Form der sulfat- und/oder nitrathaltigen Eisen(III)-salz-Lösung zuzusetzen.

Am meisten bevorzugt verwendet man im Schritt a) Natriumhydroxid, Natriumcarbonat und/oder Natriumbicarbonat als Base, bevorzugt in Form ihrer wässrigen Lösungen.

Die Umsetzung mit der Base wird bevorzugt nicht bei erhöhten Temperaturen durchgeführt, da diese zu einer beschleunigten Alterung des gebildeten Hydroxids führen könnten. Bevorzugt hält man die Temperatur bei der Umsetzung im Bereich von 10 bis 40 °C, bevorzugter von 20 bis 30 °C, noch bevorzugter führt man die Umsetzung bei Raumtemperatur (25°C) durch. Man kann die Suspension nach der Fällung zweckmäßig etwas ruhen lassen. In der Praxis kann die Suspension beispielsweise während 1 bis 5 Stunden bei Raumtemperatur oder darunter stehen gelassen werden. Die Suspension kann dabei gerührt werden.

Bevorzugt wird der erhaltene Niederschlag anschließend ein-, bevorzugt mehrmals mit Wasser gewaschen, wobei das Wasser nach dem Waschen/Aufschlämmen jeweils bevorzugt durch Abdekantieren, Abfiltrieren, Zentrifugieren und/oder durch umkehrosmotische Verfahren, wie durch Membranfiltration entfernt wird. Das erhaltene feuchte Produkt wird nicht zur Trocknung gebracht. Das feuchte Produkt wird in Wasser aufgeschlämmt. Die Wassermenge ist nicht kritisch; bevorzugt wird so gearbeitet, dass der Eisengehalt der erhaltenen Suspension (berechnet als Fe) bis zu 10 Gewichtsprozent, besonders bevorzugt 2 bis 8 Gewichtsprozent beträgt.

Die erhaltene wässrige Suspension des Eisenhydroxids weist bevorzugt einen annähernd neutralen pH-Wert im Bereich von etwa 6,5 bis 7,5 auf, bevor sie mit dem weiteren Bestandteil versetzt wird. Niedrigere pH-Werte würden dazu führen, dass das Eisenhydroxid partiell wieder in Lösung geht. Höhere pH-Werte sind unerwünscht, da sie im Schritt c) zur Komplexbildung führen können.

Besonders bevorzugt wird das erfindungsgemäße Verfahren so durchgeführt, dass vor der Zugabe des Weiteren Bestandteils im Schritt c) im Wesentlichen keine Alterung des Eisenhydroxids stattgefunden hat. Bei der Alterung von Niederschlägen erfolgt vielfach die Umgruppierung von zunächst regellos gelagerten Molekülen unter Aufbau eines mehr oder weniger regelmäßigen Kristallgitters. Bei der Alterung von Niederschlägen tritt meist nicht nur Kristallisation, sondern auch eine Teilchenvergrößerung infolge von Ostwald-Reifung auf.

Zu der oben erhaltenen Suspension wird im Schritt c) mindestens ein weiterer Bestandteil zugegeben, der die vorstehend beschriebene Alterung des in Schritt b) erhaltenen Niederschlags von Eisenhydroxid inhibiert. Dieser die Alterung des Eisenhydroxids inhibierende Bestandteil wird aus der Gruppe ausgewählt, die aus Kohlenhydraten, und Humussäure besteht. Der Bestandteil wird bevorzugt in fester Form zugegeben, prinzipiell ist jedoch auch die Zugabe in Form einer wässrigen Lösung möglich.

Besonders bevorzugt werden erfindungsgemäß Kohlehydrate als weiterer die Alterung inhibierender Bestandteil verwendet, wie verschiedene Kohlenhydrate und Zucker, z.B. Agarose, Dextran, Dextrin, Maltodextrin, Dextrinderivate, Dextranderivate, Stärke, Zellulose, wie mikrokristalline Zellulose und Zellulosederivate, Saccharose, Maltose, Lactose oder Mannit.

Besonders bevorzugt sind Stärke, Saccharose, Dextrin und/oder eine Mischung davon. Am meisten bevorzugt sind Stärke, Saccharose oder eine Mischung davon. Sehr bevorzugt ist eine Mischung von Saccharose und mindestens einem weiteren Bestandteil, der insbesondere ausgewählt wird aus Stärke, Maltodextrinen und Zellulose, insbesondere mikrokristalline Zellulose. Die Funktion des zusätzlichen Bestandteils ist vermutlich - ohne an eine Theorie gebunden zu sein - in einer Stabilisierung des frisch gefällten Eisenhydroxids zu sehen, wodurch eine Alterung des Eisenhydroxid-Niederschlags verhindert wird.

Es ist bevorzugt, die Menge an Kohlenhydraten bzw. Humussäure so zu wählen, dass mindestens 0,5 g, bevorzugt mindestens 1 g des Weiteren, die Alterung des Eisenhydroxids inhibierenden Bestandteils, d.h. Kohlenhydrat und/oder Humussäure pro g Eisen (berechnet als Fe) zugesetzt werden. Der Eisengehalt der erhaltenen Zusammensetzung beträgt maximal 50, bevorzugt maximal etwa 40 Gewichtsprozent. Der Eisengehalt der erhaltenen Zusammensetzung beträgt bevorzugt mindestens 20 Gew.-% betragen. Der maximale Gehalt des die Alterung des Eisenhydroxids inhibierenden Bestandteils, d.h. Kohlenhydrate und/oder Humussäure, unterliegt keiner Begrenzung und wird in erster Linie durch wirtschaftliche Gründe bestimmt. Bevorzugt liegt der genannte Gehalt bei etwa 5 bis 60 Gew.-%, bevorzugter bei etwa 20 bis 60 Gew.-%.

Nach der Zugabe des die Alterung des Eisenhydroxids inhibierenden Bestandteils in Schritt c) wird die erhaltene wässrige Suspension in an sich bekannter Weise getrocknet. Die Trocknung kann beispielweise durch Einengen im Vakuum oder durch Sprühtrocknung erfolgen.

In einer bevorzugten Ausführungsform des erfindungsgemäßen Verfahrens setzt man vor oder nach der Trocknung der erfindungsgemäß erhaltenen Zusammensetzung mindestens ein Calciumsalz zu. Als Calciumsalze kommen beispielsweise Salze anorganischer oder organischer Säuren, insbesondere Calciumacetat, in Frage. Durch die Zugabe des Calciumsalzes wird das Phosphatbindungsvermögen erhöht, insbesondere bei höheren pH-Werten. Es ist besonders günstig, solche mit Calciumsalzen versehene Adsorbentien bei pH-Werten von mehr als 5 einzusetzen, da selbst dann das vollständige Phosphatbindungsvermögen beibehalten bleibt. Es hat sich gezeigt, dass eine Zugabe von 400 mg bis 2 g, beispielsweise etwa 1 g Calciumsalz, insbesondere Calciumacetat pro g Eisen besonders günstig ist.

Bei dem erfindungsgemäß erhaltenen Material handelt es sich im Wesentlichen um eine physikalische Mischung von Eisenhydroxid und dem die Alterung des Eisenhydroxids inhibierenden Bestandteils, d.h. Kohlehydrate oder Humussäure. Wie oben bereits erwähnt, wird angenommen, dass letztere mit dem frisch gefällten Eisenhydroxid in Kontakt treten und für eine Stabilisierung des Hydroxids führen, so dass keine die Phosphatadsorptionsfähigkeit einschränkende Alterung des Materials auftritt. Eine Komplexbildung, wie sie in der DE 42 39 442 beschrieben ist, kann unter den erfindungsgemäß gewählten Bedingungen des Versetzens mit einer wässrigen Suspension nicht eintreten, da die Komplexbildung stark alkalische Bedingungen beim Versetzen des Eisenhydroxids mit beispielsweise Kohlehydraten erfordert.

Die nach dem erfindungsgemäßen Verfahren erhaltenen Zusammensetzungen werden bevorzugt zur Herstellung eines Adsorbens für Phosphat aus wässrigen Lösungen verwendet. Bevorzugt wird aus den nach dem erfindungsgemäßen Verfahren erhaltenen Zusammensetzungen ein Präparat zur oralen und/oder parenteralen Verabreichung bei Menschen oder Tieren hergestellt. Besonders werden die erfindungsgemäß erhaltenen Zusammensetzungen zur Herstellung eines Präparates zur Prophylaxe und/oder Behandlung des hyperphosphatämischen Zustands verwendet. Besonders bevorzugt werden die erfindungsgemäß erhaltenen Zusammensetzungen zur Herstellung eines Präparates zur Prophylaxe und/oder Behandlung von Dialyse-Patienten verwendet.

Die erfindungsgemäß erhaltenen Zusammensetzungen werden dazu in an sich bekannter Weise in pharmazeutische Darreichungsformen formuliert, wie beispielsweise zur oralen Anwendung. Sie können als solche oder zusammen mit üblichen Arzneimittelzusätzen, wie üblichen Trägern oder Hilfsmitteln, formuliert werden. Beispielsweise kann eine Einkapselung erfolgen, wobei als Einkapselungsmittel übliche auf dem pharmazeutischen Sektor verwendete Materialien, wie Hart- oder Weichgelatinekapseln eingesetzt werden können. Auch ist eine Mikroverkapselung der erfindungsgemäß erhaltenen Zusammensetzungen möglich. Es ist auch möglich, die Adsorbentien gegebenenfalls zusammen mit Hilfs- und Zusatzstoffen als Granulate, Tabletten, Dragees, in Sachets abgefüllt, als Gel oder als Sticks bereitzustellen. Die Tagesdosis der erfindungsgemäß erhaltenen Zusammensetzungen liegt beispielsweise bei 1 bis 3 g, vorzugsweise bei etwa 1,5 g bezogen auf Eisen.

Die erfindungsgemäß erhaltenen Zusammensetzungen sind auch geeignet zur Verwendung zur Adsorption von nahrungsmittelgebundenem Phosphat; zu diesem Zweck können sie beispielsweise Nahrungsmitteln beigemischt werden. Hierzu können beispielsweise Formulierungen erstellt werden, wie sie vorstehend für Arzneimittel beschrieben wurden.

Die erfindungsgemäß erhaltenen Zusammensetzungen sind insbesondere als Adsorbens insbesondere für anorganisches und nahrungsmittelgebundenes Phosphat aus Körperflüssigkeiten, Speisebrei und Nahrungsmitteln geeignet. Sie weisen ein ähnliches Phosphatadsorptionsvermögen wie die gemäß EP 0 868 125 erhaltenen Mittel auf und sind einfach und kostengünstig herstellbar.

Die Erfindung betrifft weiterhin ein Adsorbens, das nach dem erfindungsgemäßen Verfahren erhältlich ist.

Durch die Verwendung von Eisensulfat und/oder Eisennitrat als Ausgangsmaterial kann eine Zusammensetzung erhalten werden, die einen besonders niedrigen Gehalt an Chlorid aufweist, dass nur in Spuren in der Zusammensetzung enthalten ist. Der Chloridgehalt ist insbesondere niedriger als der für Akageneit übliche Chloridgehalt. Offenbart wird somit auch eine Zusammensetzung, enthaltend Eisen(III)-hydroxid sowie mindestens einen Bestandteil der aus der Gruppe ausgewählt wird, die aus Kohlehydraten und Humussäuren besteht, die weniger als 0,05, bevorzugt weniger als 0,03, noch bevorzugter weniger als 0,01 Gew.-% Chlorid enthält.

Die Erfindung wird durch die folgenden Beispiele näher erläutert:

### Beispiel 1

Zu 1160 g Sodalösung (d²⁰ = 1,185 g/ml) werden im Verlauf von 20 - 30 min unter Rühren (Flügelrührer) 444 g Eisen(III)-sulfatlösung (11,3 % m/m Fe) zugetropft. Die Suspension wird während einer Stunde weitergerührt. Daraufhin wird die Suspension unter Rühren mit 2 Liter Wasser versetzt, stehen gelassen und dann die überstehende Lösung abdekantiert. Dieser Vorgang wird fünfmal wiederholt. Man erhält so 1238 g einer Suspension mit einem Eisengehalt von 4.0 % (m/m) (komplexometrisch ermittelt). Zu den 1238 g obiger Suspension werden je 73,9 g Saccharose und Stärke zugegeben. Die Suspension wird sodann am Rotationsverdampfer bei 60 °C eingeengt und im Hochvakuum bei 50 °C getrocknet. Man erhält 223 g Pulver mit einem Eisengehalt von 21,5 % (m/m).

### Bestimmung des Phosphatadsorptionsvermögens:

Zu 233 mg des gemäß obigem Beispiel hergestellten Materials (entsprechend 0.9 mmol Eisen) werden 10 ml Natriumphosphatlösung (13,68 g/l Na₃PO₄ x 12 H₂O) zugegeben (Molverhälmis Fe : P = 1 : 0.4). Nach Einstellen des pH-Wertes lässt man die Suspension 2 Stunden bei 37 °C reagieren. Danach wird die Suspension zentrifugiert, der Überstand abdekantiert, mit destilliertem Wasser auf 25 ml aufgefüllt und dessen Phosphorgehalt bestimmt. Die Phosphatadsorption des gemäß Beispiel hergestellten Materials, ionenchromatographisch bestimmt, betrug bei einem pH von 3,0: 0,20 mg P/mg Fe, bei einem pH von 5,5: 0,16 mg P/mgFe.

### Beispiel 2

Zu 1014 ml Natronlauge (9,6% m/v) werden im Verlauf von 20 - 30 min unter Rühren (Flügelrührer) 439 g Eisen(III)-sulfatlösung (11,5 % m/m Fe) zugetropft. Die Suspension wird während einer Stunde weitergerührt. Daraufhin wird die Suspension unter Rühren mit 2 l Wasser versetzt, stehen gelassen und dann die überstehende Lösung abdekantiert. Dieser Vorgang wird wiederholt, bis der abdekantierte Überstand sulfatfrei ist (Kontrolle mit Bariumchlorid). Man erhält so 1606 g einer Suspension mit einem Eisengehalt von 2,74 % (m/m) (komplexometrisch ermittelt). Zu den 1606 g obiger Suspension werden je 66,0 g Saccharose und Stärke zugegeben. Die Suspension wird sodann am Rotationsverdampfer bei 60 °C eingeengt und im Hochvakuum bei 50 °C getrocknet. Man erhält 190 g Pulver mit einem Eisengehalt von 22,2 % (m/m).

### Bestimmung des Phosphatadsorptionsvermögens:

Zu 226 mg des gemäß Beispiel 2 hergestellten Materials (entsprechend 0.9 mmol Eisen) werden 10 ml Natriumphosphatlösung (13,68 g/l Na₃PO₄ x 12 H₂O) zugegeben (Molverhältnis Fe : P = 1 : 0.4). Nach Einstellen des pH-Wertes lässt man die Suspension 2 Stunden bei 37 °C reagieren. Danach wird die Suspension zentrifugiert, der Überstand abdekantiert, mit dest. Wasser auf 25 ml aufgefüllt und dessen Phosphorgehalt bestimmt.

Die Phosphatadsorption des gemäss Beispiel 1 hergestellten Materials, ionenchromatographisch bestimmt, betrug bei einem pH von 3,0: 0,19 mg P/mg Fe, bei einem pH von 5,5: 0,15 mg P/mg Fe.

### Beispiel 3

Zu 1200 g Sodalösung (d²⁰ = 1,185 g/ml) werden im Verlauf von 20 - 30 min unter Rühren (Flügelrührer) 535 g Eisen(III)-nitratlösung (9,7 % m/m Fe) zugetropft. Die Suspension wird während einer Stunde weitergerührt. Daraufhin wird die Suspension in einen Filtersack überführt und durch kontinuierliches Spülen mit Wasser während 3 Stunden gewaschen (Leitfähigkeit des Waschwassers nach 3 Stunden ca. 300 µS/cm). Man erhält so 923 g einer Suspension mit einem Eisengehalt von 4,3 % (m/m) (komplexometrisch ermittelt). Zu den 923 g obiger Suspension werden je 60,1 g Saccharose und Stärke zugegeben. Die Suspension wird sodann am Rotationsverdampfer bei 60 °C eingeengt und im Hochvakuum bei 50 °C getrocknet. Man erhält 172 g Pulver mit einem Eisengehalt von 22,3 % (m/m).

### Bestimmung des Phosphatadsorptionsvermögens:

Zu 225 mg des gemäß Beispiel 3 hergestellten Materials (entsprechend 0,9 mmol Eisen) werden 10 ml Natriumphosphatlösung (13,68 g/l Na₃PO₄ x 12 H₂O) zugegeben (Molverhältnis Fe : P = 1 : 0.4). Nach Einstellen des pH-Wertes lässt man die Suspension 2 Stunden bei 37 °C reagieren. Danach wird die Suspension zentrifugiert, der Überstand abdekantiert, mit dest. Wasser auf 25 ml aufgefüllt und dessen Phosphorgehalt bestimmt.
Die Phosphatadsorption des gemäss Beispiel hergestellten Materials, ionenchromatographisch bestimmt, betrug bei einem pH von 3,0: 0,21 mg P/mg Fe, bei einem pH von 5,5: 0,17 mg P/mg Fe.

### Beispiel 4

Zu 615 g Sodalösung (d²⁰ = 1,185 g/ml) werden im Verlauf von 20 - 30 min unter Rühren (Flügelrührer) 234 g Eisen(III)-sulfatlösung (11,4 % m/m Fe) zugetropft. Die Suspension wird während einer Stunde weitergerührt. Daraufhin wird die Suspension in einen Filtersack überführt und durch kontinuierliches Spülen mit Wasser während ca. 3 Stunden gewaschen (Test auf Sulfatabwesenheit mit Bariumchlorid).

Man erhält so 470 g einer Suspension mit einem Eisengehalt von 6,0 % (m/m) (komplexometrisch ermittelt). Zu den 470 g obiger Suspension werden je 21,1 g Saccharose und Maltodextrin zugegeben. Die Suspension wird sodann am Rotationsverdampfer bei 60 °C eingeengt und im Hochvakuum bei 50 °C getrocknet. Man erhält 66 g Pulver mit einem Eisengehalt von 20,3 % (m/m).

### Bestimmung des Phosphatadsorptionsvermögens:

Zu 247 mg des gemäß Beispiel 4 hergestellten Materials (entsprechend 0.9 mmol Eisen) werden 10 ml Natriumphosphatlösung (13,68 g/l Na₃PO₄ x 12 H₂O) zugegeben (Molverhältnis Fe : P = 1 : 0.4). Nach Einstellen des pH-Wertes lässt man die Suspension 2 Stunden bei 37 °C reagieren. Danach wird die Suspension zentrifugiert, der Überstand abdekantiert, mit dest. Wasser auf 25 ml aufgefüllt und dessen Phosphorgehalt bestimmt.
Die Phosphatadsorption des gemäß Beispiel 5 hergestellten Materials, ionenchromatographisch bestimmt, betrug bei einem pH von 3,0: 0,21 mg P/mg Fe, bei einem pH von 5,5: 0,17 mg P/mg Fe.

### Beispiel 5

Zu 585 g Sodalösung (d²⁰ = 1,185 g/ml) werden im Verlauf von 20 - 30 min unter Rühren (Flügelrührer) 223 g Eisen(III)-sulfatlösung (11,3 % m/m Fe) zugetropft. Die Suspension wird während einer Stunde weitergerührt. Daraufhin wird die Suspension in einen Filtersack überführt und durch kontinuierliches Spülen mit Wasser während ca. 3 Stunden gewaschen (Test auf Sulfatabwesenheit mit Bariumchlorid). Man erhält so 447 g einer Suspension mit einem Eisengehalt von 6,0 % (m/m) (komplexometrisch ermittelt). Zu den 447 g obiger Suspension werden je 20,6 g Saccharose und kristalline Zellulose zugegeben. Die Suspension wird sodann am Rotationsverdampfer bei 60 °C eingeengt und im Hochvakuum bei 50 °C getrocknet. Man erhält 65 g Pulver mit einem Eisengehalt von 20,6 % (m/m).

### Bestimmung des Phosphatadsorptionsvermögens:

Zu 244 mg des gemäß Beispiel 5 hergestellten Materials (entsprechend 0,9 mmol Eisen) werden 10 ml Natriumphosphatlösung (13,68 g/l Na₃PO₄ x 12 H₂O) zugegeben (Molverhältnis Fe : P = 1 : 0.4). Nach Einstellen des pH-Wertes lässt man die Suspension 2 Stunden bei 37 °C reagieren. Danach wird die Suspension zentrifugiert, der Überstand abdekantiert, mit dest. Wasser auf 25 ml aufgefüllt und dessen Phosphorgehalt bestimmt.
Die Phosphatadsorption des gemäß Beispiel 5 hergestellten Materials, ionenchromatographisch bestimmt, betrug bei einem pH von 3,0: 0,20 mg P/mg Fe, bei einem pH von 5,5: 0,17 mg P/mg Fe.

## Patentansprüche

1. Verfahren zur Herstellung einer Zusammensetzung, die weniger als 0,05 Gew.-% Chlorid und mindestens 20 Gew.-% aber nicht mehr als 50 Gew.-% Eisen enthält, das die folgenden Schritte umfasst:
a) Versetzen einer wässrigen, sulfat- und/oder nitrathaltigen Eisen(III)-salz-Lösung mit mindestens einer Base unter Bildung eines Niederschlags von Eisenhydroxid, wobei ein pH-Wert von mindestens 3 eingestellt wird,
b) Gegebenenfalls ein- oder mehrmaliges Waschen des erhaltenen Niederschlags mit Wasser, wobei eine wässrige Suspension des Eisenhydroxids erhalten wird,
c) Versetzen der erhaltenen wässrigen Suspension mit mindestens einem weiteren Bestandteil, ausgewählt aus Kohlenhydraten und Humussäure, der die Alterung des in Schritt b) erhaltenen Niederschlags von Eisenhydroxid inhibiert,
d) Trocknen der im Schritt c) erhaltenen Zusammensetzung.

2. Verfahren nach Anspruch 1, **dadurch gekennzeichnet, dass** eine sulfathaltige Eisen(III)-salz-Lösung verwendet wird.

3. Verfahren nach einem der Ansprüche 1 und 2, **dadurch gekennzeichnet, dass** die Eisen(III)-salz-Lösung eine wässrige Lösung von Eisen(III)-sulfat ist.

4. Verfahren nach einem der Ansprüche 1 bis 3, **dadurch gekennzeichnet, dass** die Eisen(III)-salz-Lösung eine wässrige Lösung von Eisen(III)-nitrat ist.

5. Verfahren nach einem der Ansprüche 1 bis 4, **dadurch gekennzeichnet, dass** die Base aus Alkalimetall- und Erdalkalimetall-Verbindungen ausgewählt wird.

6. Verfahren nach einem der Ansprüche 1 bis 5, **dadurch gekennzeichnet, dass** die Base aus Alkalimetall-Hydroxiden und AlkalimetallCarbonaten ausgewählt wird.

7. Verfahren nach einem der Ansprüche 1 bis 6, **dadurch gekennzeichnet, dass** man im Schritt a) eine wässrige Lösung eines Alkalimetallcarbonats, eines Alkalimetallbicarbonats oder eines Alkalimetallhydroxids als Base verwendet.

8. Verfahren nach einem der Ansprüche 1 bis 7, **dadurch gekennzeichnet, dass** man im Schritt a) einen pH-Wert von mindestens 6 einstellt.

9. Verfahren nach einem der Ansprüche 1 bis 8, **dadurch gekennzeichnet, dass** im Schritt a) Natriumhydroxid, Natriumcarbonat und/oder Natriumbicarbonat als Base verwendet wird.

10. Verfahren nach einem der Ansprüche 1 bis 9, **dadurch gekennzeichnet, dass** der die Alterung des Eisenhydroxids inhibierende weitere Bestandteil aus der Gruppe ausgewählt wird, die aus Stärke, Saccharose und Dextrin besteht.

11. Verfahren nach einem der Ansprüche 1 bis 10, **dadurch gekennzeichnet, dass** man vor oder nach dem Trocknen der Zusammensetzung mindestens ein Calciumsalz zusetzt.

12. Verfahren nach einem der Ansprüche 1 bis 11, **dadurch gekennzeichnet, dass** vor der Zugabe des weiteren Bestandteils im Schritt c) im wesentlichen keine Alterung des Eisenhydroxids stattgefunden hat.

13. Verfahren nach einem der Ansprüche 1 bis 12, **dadurch gekennzeichnet, dass** die erhaltene Zusammensetzung in einem weiteren Schritt e) in eine pharmazeutische Darreichungsform formuliert wird.

14. Verwendung der Zusammensetzung, die nach einem der Ansprüche 1 bis 13 erhältlich ist, zur Herstellung eines Adsorbens für Phosphat aus wässrigen Lösungen.

15. Verwendung der Zusammensetzung, die nach einem der Ansprüche 1 bis 13 erhältlich ist, zur Herstellung eines Präparates zur oralen und/oder parenteralen Verabreichung bei Menschen oder Tieren.

16. Verwendung der Zusammensetzung, die nach einem der Ansprüche 1 bis 13 erhältlich ist, zur Herstellung eines Präparates zur Prophylaxe und/oder Behandlung des hyperphosphatämischen Zustands.

17. Verwendung nach einem der Ansprüche 14 bis 16 zur Herstellung eines Präparates zur prophylaktischen und/oder therapeutischen Behandlung von Dialyse-Patienten.

18. Phosphat-Adsorbens, erhältlich nach dem Verfahren nach einem der Ansprüche 1 bis 13.

19. Verwendung der Zusammensetzung, die nach einem der Ansprüche 1 bis 13 erhalten wurde, zur Herstellung eines Phosphat-Adsorbens zur Beimischung zu Nahrungsmitteln.

## Claims

1. A method for producing a composition which contains less than 0.05 % by weight chloride and at least 20 % by weight, but no more than 50 % by weight iron, the method comprising the following steps:
a) mixing an aqueous, sulphate and/or nitrate bearing iron-III-salt solution with at least one base while forming a precipitate of iron hydroxide, wherein a pH value of at least 3 is set,
b) where applicable, one or more times washing the obtained precipitate with water, wherein an aqueous suspension of the iron hydroxide will be obtained,
c) mixing the obtained aqueous suspension with at least one more component selected from carbohydrates and ulmic acid, which component inhibits the ageing of the precipitate of iron hydroxide obtained in step b),
d) drying the composition obtained in step c).

2. A method according to claim 1, **characterized in that** a sulphate bearing iron-III-salt solution will be used.

3. A method according to one of the claims 1 and 2, **characterized in that** the iron-III-salt solution is an aqueous solution of iron-III-sulphate.

4. A method according to one of the claims 1 through 3, **characterized in that** the iron-III-salt solution is an aqueous solution of iron-III-nitrate.

5. A method according to one of the claims 1 through 4, **characterized in that** the base will be selected from alkali metal and alkaline earth metal compounds.

6. A method according to one of the claims 1 through 5, **characterized in that** the base will be selected from alkali metal hydroxides and alkali metal carbonates.

7. A method according to one of the claims 1 through 6, **characterized in that** in step a) an aqueous solution of an alkali metal carbonate, an alkali metal bicarbonate or an alkali metal hydroxide will be used as base.

8. A method according to one of the claims 1 through 7, **characterized in that** in step a) a pH value of at least 6 will be set.

9. A method according to one of the claims 1 through 8, **characterized in that** in step a) sodium hydroxide, sodium carbonate and/or sodium bicarbonate will be used as base.

10. A method according to one of the claims 1 through 9, **characterized in that** the other component which inhibits the ageing of the iron hydroxide will be selected from the group composed of starch, sucrose and dextrin.

11. A method according to one of the claims 1 through 10, **characterized in that** at least one calcium salt will be added before or after the drying of the composition.

12. A method according to one of the claims 1 through 11, **characterized in that** before the addition of the other component in step c), no ageing of the iron hydroxide has essentially taken place.

13. A method according to one of the claims 1 through 12, **characterized in that** the obtained composition will be formulated in another step e) as a pharmaceutical form.

14. A use of the composition that can be obtained according to one of the claims 1 through 13 for producing an adsorbent of phosphate in aqueous solutions.

15. A use of the composition that can be obtained according to one of the claims 1 through 13 for producing a preparation for the oral and/or parenteral administration to humans or animals.

16. A use of the composition that can be obtained according to one of the claims 1 through 13 for producing a preparation for the prophylaxis and/or the treatment of the hyperphosphatemic condition.

17. A use according to one of the claims 14 through 16 for producing a preparation for the prophylactic and/or therapeutic treatment of dialysis patients.

18. A phosphate adsorbent that can be obtained according to the method according to one of the claims 1 through 13.

19. A use of the composition that can be obtained according to one of the claims 1 through 13 for producing a phosphate adsorbent for the admixture to food products.

## Revendications

1. Procédé de fabrication d'une composition, qui contient moins de 0,05 % en poids de chlorure et au moins 20 % en poids, mais pas plus de 50 % en poids de fer, le procédé comprenant les étapes suivantes de:
a) mélanger une solution de sel de fer III aqueuse et contenant du sulfate et/ou du nitrate avec au moins une base en formant une précipitation d'hydroxyde de fer, une valeur de pH d'au moins 3 étant fixée,
b) le cas échéant, laver une fois ou plusieurs fois la précipitation obtenue avec de l'eau, une suspension aqueuse de l'hydroxyde de fer étant obtenue,
c) mélanger la suspension aqueuse obtenue avec au moins un autre composant sélectionné parmi des hydrates de carbone et l'acide humique, lequel composant inhibe le vieillissement de la précipitation d'hydroxyde de fer obtenue dans l'étape b),
d) sécher la composition obtenue dans l'étape c).

2. Procédé selon la revendication 1, **caractérisé en ce qu'**on utilise une solution de sel de fer III contenant du sulfate.

3. Procédé selon l'une des revendications 1 et 2, **caractérisé en ce que** la solution de sel de fer III est une solution aqueuse de sulfate de fer III.

4. Procédé selon l'une des revendications 1 à 3, **caractérisé en ce que** la solution de sel de fer III est une solution aqueuse de nitrate de fer III.

5. Procédé selon l'une des revendications 1 à 4, **caractérisé en ce que** la base est sélectionnée parmi des composés de métal alcalin et de métal alcalinoterreux.

6. Procédé selon l'une des revendications 1 à 5, **caractérisé en ce que** la base est sélectionnée parmi des hydroxydes de métal alcalin et des carbonates de métal alcalin.

7. Procédé selon l'une des revendications 1 à 6, **caractérisé en ce qu'**on utilise une solution aqueuse d'un carbonate de métal alcalin, d'un bicarbonate de métal alcalin ou d'un hydroxyde de métal alcalin dans l'étape a).

8. Procédé selon l'une des revendications 1 à 7, **caractérisé en ce qu'**on fixe une valeur de pH d'au moins 6 dans l'étape a).

9. Procédé selon l'une des revendications 1 à 8, **caractérisé en ce qu'**on utilise de l'hydroxyde de sodium, du carbonate de sodium et/ou du bicarbonate de sodium comme base dans l'étape a).

10. Procédé selon l'une des revendications 1 à 9, **caractérisé en ce que** l'autre composant, qui inhibe le vieillissement de l'hydroxyde de fer est sélectionné dans le groupe composé d'amidon, de saccharose et de dextrine.

11. Procédé selon l'une des revendications 1 à 10, **caractérisé en ce qu'**on ajoute au moins un sel de calcium avant ou après le séchage de la composition.

12. Procédé selon l'une des revendications 1 à 11, **caractérisé en ce qu'**avant l'addition d'un autre composant dans l'étape c), un vieillissement de l'hydroxyde de fer n'a pas essentiellement tenu lieu.

13. Procédé selon l'une des revendications 1 à 12, **caractérisé en ce que** la composition obtenue est formulée dans une forme pharmaceutique dans une autre étape e).

14. Utilisation de la composition qu'on obtient selon l'une des revendications 1 à 13 pour fabriquer un adsorbant de phosphate dans des solutions aqueuses.

15. Utilisation de la composition qu'on obtient selon l'une des revendications 1 à 13 pour fabriquer une préparation destinée à l'administration orale et/ou parentérale chez les hommes ou les animaux.

16. Utilisation de la composition qu'on obtient selon l'une des revendications 1 à 13 pour fabriquer une préparation destinée à la prophylaxie et/ou au traitement de l'état hyper-phosphatémique.

17. Utilisation selon l'une des revendications 14 à 16 pour fabriquer une préparation destinée au traitement prophylactique et/ou thérapeutique des patients de dialyse.

18. Adsorbant de phosphate qu'on obtient selon le procédé selon l'une des revendications 1 à 13.

19. Utilisation de la composition qu'on obtient selon l'une des revendications 1 à 13 pour fabriquer un adsorbant de phosphate pour l'addition à des produits alimentaires.
